Europäisches Patentamt

(19) European Patent Office

Office européen des brevets

(11) Numéro de publication: **0 145 596**
**B1**

(12) # FASCICULE DE BREVET EUROPÉEN

(45) Date de publication du fascicule du brevet:
05.11.86

(51) Int. Cl.⁴: **C 07 C 45/63, C 07 C 47/14**

(21) Numéro de dépôt: **84402516.3**

(22) Date de dépôt: **06.12.84**

(54) Procédé catalytique de préparation de trifluoroacétaldéhyde.

(30) Priorité: 13.12.83 FR 8319914

(43) Date de publication de la demande:
19.06.85 Bulletin 85/25

(45) Mention de la délivrance du brevet:
05.11.86 Bulletin 86/45

(84) Etats contractants désignés:
DE FR GB IT NL

(56) Documents cité:
FR-A-88 728
FR-A-1 452 159
FR-A-2 034 585
US-A-3 017 436

Le dossier contient des informations techniques
présentées postérieurement au dépôt de la
demande et ne figurant pas dans le présent
fascicule.

(73) Titulaire: ATOCHEM, 4 & 8, Cours Michelet La
Défense 10, F-92800 Puteaux (FR)

(72) Inventeur: Cheminal, Bernard Rés. Beauséjour
Saint- Irénée, 26, rue Soeur Bouvier, F-69005 Lyon
(FR)
Inventeur: Mathais, Henri, Villa No. 2 Chemin de
l'Indiennerie, F-69370 Saint- Didier au Mont d'Or
(FR)

(74) Mandataire: Leboulenger, Jean, ATOCHEM
Département Propriété Industrielle, F-92091 Paris
la Défense 10 Cédex 42 (FR)

LIBER, STOCKHOLM 1986

## Description

La présente invention concerne un procédé catalytique continu de fluoruration en phase gazeuse, par l'acide fluorhydrique anhydre, du trichloroacétaldéhyde (chloral) en trifluoroacétaldéhyde (fluoral).

La préparation, par la voie ainsi définie, du fluoral comme d'ailleurs des composés fonctionnals perfluorés en général à partir des composés perchlorés correspondants, est très difficile à réaliser.

Les difficultés auxquelles se heurte la réalisation des procédés connus sont exposées dans le brevet français n° 2 135 474: "L'état de la technique montre qu'il est difficile d'obtenir à la fois une perfluoration complète des composés perchlorés en un seul passage des réactifs dans la zone de fluoruration en phase vapeur, une productivité convenable et une perte minime de matière utile par suite des réactions parasites de décomposition en composés non utilisables".

Le brevet anglais n° 1 036 870 montre que, dans les conditions considérées comme préférées, c'est-à-dire à basse température, pour la fluoruration du chloral en fluoral sur un catalyseur $Cr_2O_3$ gel pur, le rendement en fluoral reste faible, malgré le rapport molaire très élevé HF/chloral.

Le brevet français n° 2 135 474 a lui-même pour objet un procédé-catalytique de fluoruration en phase gazeuse de composés perchlorés en composés perfluorés correspondants selon lequel le composé perfluoré visé n'est obtenu en un seul passage des réactifs sur le catalyseur qu'en réalisant la fluoruration dans trois zones de réaction successives et distinctes l'une de l'autre, la température de chacune des deux dernières zones de réaction étant réglée à une valeur supérieure de 10°C au moins à celle de la température de la zone de réaction qui la précède.

Une telle façon de procéder suppose une technologie complexe, délicate et coûteuse. De plus, la quantité pratique d'acide fluorhydrique, qui est de 1,74 fois la quantité molaire stoéchiométrique dans le cas de la fluoruration du chloral, rend nécessaire, pour l'économie du procédé, une importante installation de récupération et de recyclage de l'acide fluorhydrique non transformé.

Dans le brevet US 3 017 436 qui concerne la fluoruration du chloral par l'acide fluorhydrique dans un rapport molaire compris entre 3 et 18 et à une température comprise entre 200 et 400°C en présence d'un catalyseur à base d'halogénure de cobalt, nickel ou chrome sur une alumine, le catalyseur est activé au moyen d'aon l'exemple unique, le rendement en fluoral n'atteint que 70 %.

Le brevet FR 2 034 585 relatif à l'hydrofluoration d'hydrocarbures halogénés, pouvant éventuellement renfermer un groupe carbonyle, avec du gaz fluorhydrique en excès à des températures comprises entre 140 et 400°C préconise l'emploi d'un catalyseur constitué d'alumine-$\gamma$ ou -$\eta$ contenant du chrome, de l'oxygène et du fluor. Ce catalyseur est activé avec de l'acide fluorhydrique et de l'air à une température qui ne doit pas dépasser 250°C. Les exemples fournis décrivent l'hydrofluoration du dichloro-1,1 éthylène et du fluorure de vinyle.

Le brevet français n° 2 135 473 revendique un procédé adapté à la mise en oeuvre d'un catalyseur particulier constitué de deux composés minéraux non stoéchiométriques, l'un de chrome, l'autre de nickel. L'emploi d'un tel catalyseur, non décrit dans le brevet cité pour la fluoruration du chloral en fluoral, conduit malheureusement à une dégradation excessive des molécules organiques, à cause de réactions parasites de craquage, correspondant, en moles, à 7 à 10 % du produit à fluorer.

Le procédé selon l'invention ne présente pas les inconvénients des procédés connus et permet d'obtenir une transformation élevée et sélective du chloral en fluoral en un seul passage des réactifs sur le catalyseur, dans une zone de réaction unique et avec un rapport molaire acide fluorhydrique/chloral faible.

Le procédé selon l'invention est caractérisé en ce que l'acide fluorhydrique anhydre et le chloral, dans un rapport molaire compris entre 3,3/1 et 15/1, de préférence entre 4/1 et 5/1, réagissent à une température comprise entre 230°C et 260°C sur un catalyseur constitué d'une alumine gamma imprégnée de sesquioryde de chrome $Cr_2O_3$ à raison de 1,5 à 4 et de préférence environ 2,5 atomes de chrome par litre d'alumine, et activé entre 300°C et 400°C par un mélange d'acide fluorhydrique et de trichloro-1,1,2 trifluoro-1,2,2 éthane dans un rapport molaire compris entre environ 1/2 et 2/1, de préférence égal à 1/1 environ.

Dans le procédé selon l'invention l'imprégnation de l'alumine est réalisée par des moyens connus, comme la coulée simultanée et séparée dans un réacteur maintenu en rotation sous vide et contenant l'alumine, d'une solution aqueuse d'anhydride chromique $Cr O_3$ d'une part, de méthanol d'autre part, suivie d'un séchage du produit obtenu en lit fixe ou en lit fluide, à une température en général voisine de 150°C sous courant de gaz inerte tel que l'azote.

La réduction du chrome hexavalent en chrome trivalent peut être réalisée par un composé réducteur autre que le méthanol, par exemple un autre alcool, tel que l'éthanol, ou l'hydrazine.

L'activation du catalyseur est réalisée par exemple par pessage d'un mélange d'acide fluorhydrique et de trichloro-1,1,2-trifluoro-1,2,2-éthane sur le catalyseur séché, de préférence d'abord à 300° ou 350°C, puis à 400°C.

La pression absolue à laquelle est effectuée la fluoruration du chloral est généralement de 1 bar environ.

Le débit des réactifs gazeux sur le catalyseur est le plus souvent compris entre 150 et 250 l/h/kg de catalyseur.

Les exemples suivants, donnés à titre non

## EXEMPLE 1

Un catalyseur $Cr_2O_3$ sur alumine gamma est préparé da la manière suivante: dans un réacteur sphérique maintenu en rotation et sous vide, contenant 0,4 1 d'alumine gamma commercialisée par la Société Harshaw sous la référence Al 111 73 E, dont les principales caractéristiques sont :
- présentation : extrudés de 0,8 mm de diamètre environ
 - teneur en $SiO_2$ : 0,13 %
 - surface spécifique totale : 161 m2/g
 - rayon moyen de pore : 105,9 Å
 - volume total de pore : 0,91 ml/g
 - pourcentage de pores compris entre 150 et 250 Å : 16,4
 - pourcentage de pores compris entre 250 et 300 Å : 1,7
 sont coulées simultanément et séparément, en 1 heure environ, d'une part, 190 g d'une solution aqueuse contenant de l'anhydride chromique $CrO_3$ à une concentration en poids de 52,6 %, d'autre part un mélange de méthanol et d'eau contenant 80 % en poids de méthanol.
 L'alumine ainsi imprégnée est ensuite séchée en lit fluide à 150° C sous un courant d'azote.
 Une quantité égale à 0,189 kg de catalyseur sec est activée dans un réacteur tubulaire de fluoruration de 40 mm de diamètre, d'abord à 350° C durant 6 heures puis à 400° C durant 24 heures par passage de 13,5 Nl/h d'un mélange gazeux équimoléculaire d'acide fluorhydrique et de trichloro-1,1,2 trifluoro-1,2,2- éthane.
 Sur le catalyseur ainsi activé et dans le réacteur ayant servi à l'activation mais chauffé à 250° C et sous une pression absolue de 1 bar sont ensuite passés 38 l/h d'un mélange gazeux constitué d'acide fluorhydrique anhydre et de chloral dans un rapport molaire égal à 4,25/1.
 L'analyse de l'effluent gazeux du réacteur montre que le taux de fluoruration global par rapport à l'acide fluorhydrique, c'est-à-dire le rapport du nombre de moles d'HF consommé au nombre de moles d'HF correspondant à la stoéchiométrie de la réaction de transformation du chloral en fluoral, est de 98 %, que la conversion du chloral en fluoral est de 95,1 %, que la sélectivité en fluoral, exprimée comme étant le pourcentage de moles de fluoral formé par rapport aux moles de dérivés fluorés de l'acétaldéhyde formés, chlorés ou non, est de 98,2 % et qu'enfin la productivité atteint 0,156 kg de fluoral/h/kg de catalyseur et le taux de chloral se retrouvant sous forme dégradée par suite de réactions parasites est inférieure à 2 %.
 On obtient des résultats voisins en effectuant l'activation du catalyseur sec dans un réacteur tubulaire de 28 mm de diamètre, d'abord a 350° C durant 2 heures puis à 400° C durant 22 heures, par passage de 31,2 Nl/h d'acide fluorhydrique et 19,3 Nl/h de trichloro-1,1,2trifluoro-1,2,2 éthane.

## EXEMPLE 2

En procédant comme dans l'exemple 1 mais avec un rapport molaire HF/chloral de 4,5, le taux de fluoruration global par rapport à l'HF est de 100 %, la conversion du chloral en fluoral de 96,5 %, la sélectivité en fluoral de 98,5 % et la productivité de 0,160 kg de fluoral/h/kg de catalyseur, le taux de dégradation du chloral restant inférieur à 2 %.

## EXEMPLE 3

En procédant comme dans l'exemple 1 mais avec un rapport molaire HF/chloral de 4,70, le taux de fluoruration global par rapport à l'HF est de 100 %, la conversion du chloral en fluoral de 98,5 la sélectivité en fluoral de 98 % et la productivité de 0,158 kg de fluoral/h/kg de catalyseur, le taux de dégradation du chloral n'excédant pas 2 %.

## EXEMPLE 4

L'essai correspondant à cet exemple, effectué en procédant comme dans les exemples 1 à 3 mais avec un rapport molaire HF/chloral de 6,30, conduit à des résultats pratiquement identiques à ceux indiqués dans ces exemples et confirme que le procédé de l'invention présente l'avantage supplémentaire de procurer des résultats pratiquement insensibles à l'excès de HF par rapport à la stoéchiométrie de la réaction de transformation du chloral en fluoral lorsque cet excés varie de 30 % à 100 % environ.

## EXEMPLE 5

En procédant comme dans l'exemple 1 mais à une température de 235° C, le taux de fluoruration global par rapport à l'HF est de 77,9 %, la conversion du chloral en fluoral atteint 11,6 %, la productivité 0,12 kg de fluoral/h/kg de catalyseur, le taux de dégradation du chloral étant inférieur à 0,5 %.

## EXEMPLE 6

En procédant comme dans l'exemple 1 mais avec un rapport molaire HF/CHLORAL de 10,3, le taux de fluoruration global par rapport à l'HF est de 100 %, la conversion du chloral en fluoral est de 95,4 %, la sélectivité en fluoral de 97,8 % et la productivité de 0,082 kg de fluoral/h/kg de catalyseur, le taux de dégradation du chloral étant voisin de 2,5 %.

**EXEMPLE 7**

Un poids de 0,223 kg de catalyseur sec selon l'exemple 1 est activé dans un réacteur tubulaire de fluoruration de 28 mm de diamètre, d'abord à 350°C pendant 16 heures, puis à 400°C durant 8 heures par passage de 7,7 Nl/h d'acide fluorhydrique et 6,2 Nl/h de trichloro-1,1,2 trifluoro-1,2,2 éthane (rapport molaire = 1,24).

Le catalyseur ainsi activé est utilisé pour la fluoruration du chloral dans le même réacteur et les mêmes conditions qu'à l'exemple 1, mais avec un rapport molaire HF/Chloral de 4,3. Après 96 heures de fonctionnement, le catalyseur est régénéré à 400°C par un mélange d'air et de chlore durant 3 heures et on reprend ensuite l'opération de fluoruration. Après une heure, le taux de fluoruration global par rapport à l'HF est de 100 %, la conversion du chloral en fluoral est de 92,7 %, la sélectivité en fluoral de 98,3 % et la productivité de 0,129 kg de fluoral/h/kg de catalyseur, le taux de dégradation du chloral étant voisin de 5,7 %.

**Revendications**

1. Procédé catalytique continu de préparation de trifluoroacétaldéhyde par fluoruration du trichloroacétaldéhyde en phase gazeuse par l'acide fluorhydrique anhydré en une seule zone de réaction, caractérisé en ce que l'acide fluorhydrique et le trichloroacétaldéhyde, dans un rapport molaire compris entre 3,3 et 15 réagissent à une température comprise entre 230°C et 260°C, sur un catalyseur constitué d'une alumine gamma imprégnée de sesquioxyde de chrome $Cr_2O_3$ à raison de 1,5 à 4 atomes de chrome par litre d'alumine et activé entre 300°C et 400°C par un mélange d'acide fluorhydrique et de trichloro-1,1,2-trifluoro-1,2,2 éthane dans un rapport molaire compris entre environ 0,5 et 2.

2. Procédé selon la revendication 1 caractérisé en ce que le débit des réactifs sur le catalyseur est compris entre 150 et 250 l/h/kg de catalyseur.

3. Procédé selon la revendication 1 ou 2, dans lequel le rapport molaire acide fluorhydrique/trichloroacétaldéhyde est compris entre 4 et 5.

4. Procédé selon l'une des revendications 1 à 3, dans lequel le catalyseur contient environ 2,5 atomes de chrome par litre d'alumine et est activé par un mélange sensiblement équimolaire d'acide fluorhydrique et de trichloro-1,1,2 trifluoro-1,2,2 éthane.

**Patentansprüche**

1. Kontinuierliches katalytisches Verfahren zur Herstellung von Trifluoracetaldehyd durch Fluorierung von Trichloracetaldehyd mit wasserfreier Fluorwasserstoffsäure in der Gasphase in einer einzigen Reaktionszone, dadurch gekennzeichnet, daß die Fluorwasserstoffsäure und das Trichloracetaldehyd in einem molaren Verhältnis zwischen 3,3 und 15 bei einer Temperatur zwischen 230°C und 260°C über einen Katalysator miteinander reagieren, der aus einem Gamma-Aluminiumoxid besteht, das mit Chromsequioxid $Cr_2O_3$ in einer Menge von 1,5 bis 4 Atomen Chrom pro Liter Aluminiumoxid imprägniert und zwischen 300°C und 400°C mit einem Gemisch von Fluorwasserstoffsäure und 1,1,2-Trichlor-1,2,2-tri-fluorethan in einem molaren Verhältnis zwischen etwa 0,5 und 2 aktiviert ist.

2. Verfahren nach Anspruch 1, dadurch gekennzeichnet, daß die Einsatzmenge der Reaktionspartner über den Katalysator zwischen 150 und 250 l/h/kg Katalysator liegt.

3. Verfahren nach Anspruch 1 oder 2, bei dem das molare Verhältnis Flurwasserstoffsäure/Trichloracetaldehyd zwischen 4 und 5 liegt.

4. Verfahren nach einem der Ansprüche 1 bis 3, bei dem der Katalysator etwa 2,5 Atome Chrom pro Liter Aluminiumoxid enthält und mit einem Gemisch aktiviert wird, das im wesentlichen äquimolar an Fluorwasserstoffsäure und 1,1,2-Trichlor-1,2,2-trifluorethan ist.

**Claims**

1. Continuous catalytic process for the preparation of trifluoroacetaldehyde by gas phase fluorination of trichloroacetaldehyde by means of anhydrous hydrofluoric acid in a single reaction zone, characterized in that hydrofluoric acid and trichloroacetaldehyde, in a molar ratio of between 3.3 and 15, react at a temperature of between 230°C and 260°C on a catalyst consisting of a gamma alumina impregnated with chromium sesquioxide $Cr_2O_3$ in a proportion of 1.5 to 4 chromium atoms per litre of alumina and activated between 300°C and 400°C with a mixture of hydrofluoric acid and 1,1,2-trichloro-1,2,2-trifluoroethane in amolar ratio of between approximately 0.5 and 2.

2. Process according to Claim 1, characterized in that the flow rate of the reactants over the catalyst is between 150 and 250 l/h/kg of catalyst.

3. Process according to Claim 1 or 2, in which the molar ratio hydrofluoric acid/trichloroacetaldehyde is between 4 and 5.

4. Process according to one of Claims 1 to 3, in which the catalyst contains approximately 2.5 chromium atoms per litre of alumina and is activated with a substantially equimolar mixture of hydrofluoric acid and 1,1,2-trichloro-1,2,2-trifluoroethane.